# EUROPEAN PATENT SPECIFICATION

(11) **EP 2 604 236 B1**
(45) Date of publication and mention of the grant of the patent: **23.11.2016**
(21) Application number: 11816092.8
(22) Date of filing: 09.08.2011
(51) Int. Cl.: A61F 13/14, A61F 13/15, A41C 3/04, A41C 5/00

(54) **BREAST PAD AND PRODUCTION METHOD THEREOF**
BRUSTPOLSTER UND HERSTELLUNGSVERFAHREN DAFÜR
COUSSINET D'ALLAITEMENT ET SON PROCÉDÉ DE FABRICATION

(30) Priority: 10.08.2010 CN 201010252380
(43) Date of publication of application: 19.06.2013
(73) Proprietor: Shanghai Foliage Industry Co., Ltd, Shanghai 201711 (CN)
(72) Inventor: XIA, Shuangyin, Shanghai 201711 (CN)
(74) Representative: Witte, Weller & Partner Patentanwälte mbB
(86) International application number: PCT/CN2011/078156
(87) International publication number: WO 2012/019534

(56) References cited:
- EP-A1- 1 943 911
- CN-A- 1 907 249
- CN-A- 101 299 936
- CN-A- 101 912 163
- CN-U- 201 727 651
- CN-Y- 200 950 823
- JP-U- 59 150 437
- JP-U- 59 182 337
- JP-U- 60 017 745
- US-A- 4 892 532
- US-A- 5 843 062
- US-A- 6 074 272
- US-A- 6 074 272

## Description

This application claims priority of Chinese Patent Application No.201010252380.X, titled "BREAST PAD AND PRODUCTION METHOD THEREOF", and filed with the Chinese State Intellectual Property Office on August 10, 2010.

### FIELD OF THE INVENTION

The invention relates to the technical field of daily commodities, and more specifically, to a breast pad and a method for producing the same.

### BACKGROUND OF THE INVENTION

For lactating women, milk overflowing commonly occurs, and overflowed milk may contaminate clothing. A breast pad is a product that is put in a brassiere to absorb overflowed milk and thus to prevent clothing from being contaminated.

Generally, the current breast pad is a flat paper or cotton circular sheet or irregular-shaped sheet in appearance. Since such a breast pad is flat in appearance, multiple wrinkles may occur when it is placed in a cup of a brassiere, thus causing milk leakage.

Then, a rubber band is provided at each of two sides of the breast pad, so as to draw the two sides of the breast pad sheet in under the elasticity, being cup-shaped. However, this only can improve the milk leakage phenomenon at two sides, and milk leakage phenomenon at bottom still occurs in the case of the breast pad provided with the rubber band since overflowed milk would flow downwardly under gravity.

US 6,074,272 discloses a nursing pad liner for placing against the breast of a woman and for placing against the clothing of the woman. The liner comprises a layer of adsorbent material having an outside and an inside to adsorb and hold at least some fluid leaking from a woman's breast, and a liquid impermeable outer layer for covering the outside of the adsorbent material.

Further, EP 1 943 911 A1 discloses a breast-milk pad, which comprises a back sheet, a top sheet and an absorber that is disposed between the back sheet and the top sheet. Top sheet and back sheet are bonded to each other in an outer peripheral portion.

### SUMMARY OF THE INVENTION

In view of the fact, the present application provides a breast pad and a method for producing the same, so as to solve the problem of milk leakage as readily occurred in the existing breast pad.

In order to solve the above problem, the technical solutions are set forth herein below.

A breast pad includes an isolation layer, an absorption layer and a moisture permeable layer in sequence from bottom to top, and further includes a leakproof layer disposed on an upper surface of one end, or an upper surface of two opposite ends, or an upper surface of a periphery of the moisture permeable layer, wherein
an outer edge of the isolation layer, an outer edge of the moisture permeable layer and an outer edge of the leakproof layer are bonded by hot pressing so as to form a bonded edge, the absorption layer does not exist at the bonded edge, and a storage space is formed between the leakproof layer and the upper surface of the moisture permeable layer.

Preferably, one or two double-sided stickers are affixed to an outer surface of the isolation layer.

Preferably, the absorption layer and the isolation layer are adhered to each other by glue.

Preferably, the absorption layer is a fluffy pulp layer, or an air-laid paper layer, or the combination of a toilet paper layer and a fluffy pulp layer in sequence from bottom to top, or the combination of a toilet paper layer and a mixed layer of fluffy pulp and macromolecule absorbers in sequence from bottom to top.

Preferably, the moisture permeable layer is made of non-woven cloth.

Preferably, the leakproof layer is a plastic film layer, or a composite layer of non-woven cloth and plastic film.

Preferably, at least one guiding channel formed by hot pressing is further provided at the middle portion of the breast pad.

Preferably, the breast pad is in the shape of circle, square or triangle, or is cup-shaped.

A method for producing a breast pad, includes:
placing an isolation layer, an absorption layer and a moisture permeable layer in sequence from bottom to top, wherein outer edges of the isolation layer and moisture permeable layer extend beyond an outer edge of the absorption layer;
bonding the outer edges of the isolation layer and moisture permeable layer by hot pressing so as to form a preliminary breast pad; and
bonding outer edges of the preliminary breast pad and leakproof layer by hot pressing such that a storage space is formed by the leakproof layer and an upper surface of the moisture permeable layer, so as to form a breast pad.

Preferably, the method further includes hot-pressing on the moisture permeable layer of the breast pad to form at least one guiding channel.

As can be seen from the above technical solutions, the breast pad disclosed in the present application is of a four-layer structure. The moisture permeable layer is in direct contact with the human body, and overflowed milk flows through the moisture permeable layer and is absorbed by the absorption layer. The isolation layer isolates the brassiere from the absorption layer, so as to prevent milk from contaminating clothing. Moreover, since a storage space is formed by the leakproof layer and the upper surface of the moisture permeable layer, overflowed milk may flow through the moisture permeable layer directly into the storage space when it flows downwardly under gravity, and may be absorbed rapidly by the absorption layer without milk leakage.

Furthermore, in the breast pad disclosed in the present application, the isolation layer, the moisture permeable layer and the leakproof layer are processed by a hot-pressing bond procedure once, which is a simple operating process at low production costs, and adaptable to assembly line operation.

### BRIEF DESCRIPTION OF THE DRAWINGS

In order to explain embodiments of the present application or the technical solution in the prior art more clearly, accompanying drawings needed to be referred to in the description of the embodiments or the prior art will be briefly introduced below. Obviously, the drawings described below relate to only some embodiments of the present application, and other drawings may be occurred to the person skilled in the art on the basis of these drawings without inventive efforts.
Fig. 1 is a front view of a breast pad disclosed in an embodiment of the present application;
Fig. 2 is a side view of a breast pad disclosed in an embodiment of the present application;
Fig. 3 is a structural schematic view of a breast pad disclosed in an embodiment of the present application;
Fig. 4(a) is a structural schematic view of a breast pad disclosed in another embodiment of the present application;
Fig. 4(b) is a structural schematic view of another breast pad disclosed in another embodiment of the present application;
Fig. 4(c) is a structural schematic view of another breast pad disclosed in another embodiment of the present application;
Fig. 4(d) is a structural schematic view of another breast pad disclosed in another embodiment of the present application;
Fig. 5 is a schematic view showing the formation process of a cup-shaped breast pad disclosed in an embodiment of the present application;
Fig. 6 is a structural schematic view of a cup-shaped breast pad disclosed in an embodiment of the present application;
Fig. 7 is a flowchart of a method for producing a breast pad in embodiments of the present application.

### DETAILED DESCRIPTION OF THE INVENTION

Hereinafter, the technical solutions in the embodiments of the present application will be described clearly and fully in conjunction with the drawings of the embodiments of the present application. Apparently, the embodiments described are only a part of, not all of embodiments of the present application. Based on the embodiments of the present application, other embodiments occurred to the person skilled in the art without inventive efforts all fall into the protection scope of the present application.

The embodiments of the present application disclose a breast pad in order to solve the problem of milk leakage as readily occurred in the existing breast pad.

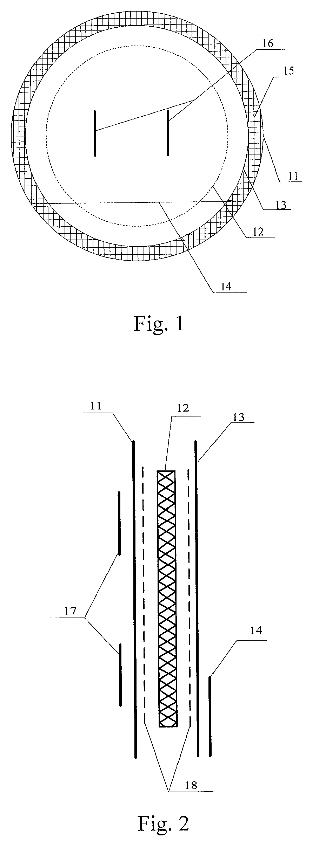

As shown in Figs. 1 and 2, a breast pad disclosed in an embodiment of the present application includes an isolation layer 11, an absorption layer 12 and a moisture permeable layer 13 which are arranged in sequence from bottom to top. A leakproof layer 14 is provided on the upper surface of one end of the moisture permeable layer 13.

Besides, the outer edge of the isolation layer 11, the outer edge of the moisture permeable layer 12 and the outer edge of a leakproof layer 14 are bonded together by hot pressing so as to form a bonded edge 15. There is no the absorption layer at the bonded edge. In addition, a storage space is formed by the leakproof layer 14 and the upper surface of the moisture permeable layer 13.

When the breast pad in the embodiment is directly placed inside a brassiere, the moisture permeable layer 13 will be in direct contact with the human body. At this moment, overflowed milk may be absorbed by the absorption layer 12 through the moisture permeable layer 13. Due to the isolation function of the isolation layer 11, milk may be effectively avoided from contaminating clothing. In addition, when milk flows down under gravity, it may be hold by the leakproof layer 14 disposed on the upper surface of the moisture permeable layer 13, temporarily stored in the storage space defined by the leakproof layer 14 and the moisture permeable layer 13, and then absorbed by the absorption layer 12, thereby avoiding the occurrence of milk leakage at the bottom.

The shape of the breast pad according to the present application may be varied, as long as it is easily placed in the brassiere. Specifically, the breast pad according to the present application may be circular, square, triangular, lace-shaped, sectorial or cup-shaped. For illustrative purpose, various embodiments are described by taking a circular breast pad as an example.

To guide the milk flow, at least one guiding channel 16 is further provided on the breast pad, and is formed by hot pressing. In this way, overflowed milk may flow along the guiding channel 16 towards the storage space defined by the leakproof layer 14 and the upper surface of the moisture permeable layer 13, and may be prevented from spreading around to cause leakage.

Furthermore, in order to effectively fix the breast pad, one or two double-sided stickers 17 are affixed on the outer surface of the isolation layer 11. In use, a stripping paper is torn off, and the breast pad is adhered to the brassiere, thereby preventing the movement of the breast pad in use. In addition, since the absorption layer 12 of the breast pad is located between the isolation layer 11 and the moisture permeable layer 13 and is not fixed, the milk absorption function of the absorption layer 12 will be influenced if it moves. As a result, the absorption layer 12 may be adhered to the isolation layer 11 and the moisture permeable layer 13 by glue, and alternatively, the isolation layer 11, the absorption layer 12 and the moisture permeable layer 13 may also be laminated as an integral piece by hot pressing.

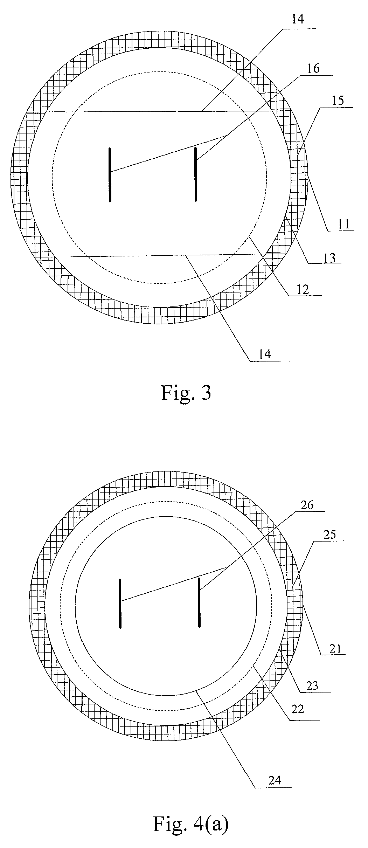

In order to avoid the fact that most of milk is absorbed by one end of the breast pad due to gravity so as to cause that one end of the breast pad cannot continue to use while the other end is unused, the breast pad disclosed in the above embodiment may be further provided with the leakproof layers 14 at two opposite ends and on the upper surface of the moisture permeable layer 13, as shown in Fig. 3. The outer edges of the two leakproof layers 14, the outer edge of the isolation layer 11 and the outer edge of the moisture permeable layer 13 form a bonded edge 15.

In this way, when one end of the breast pad has been soaked with milk, the breast pad may be inversed such that the other end works. Thus, the number of the breast pads to be used may be reduced. Further, the leakproof layers 14 arranged at both ends may also solve the problem that milk of a user of the breast pad would flow to the upper end of the breast pad to contaminate clothing during movement.

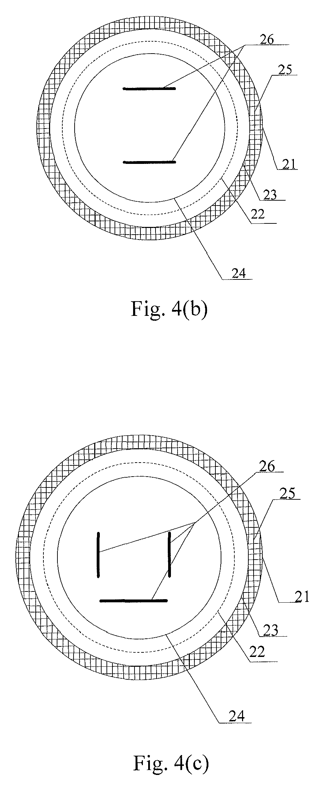

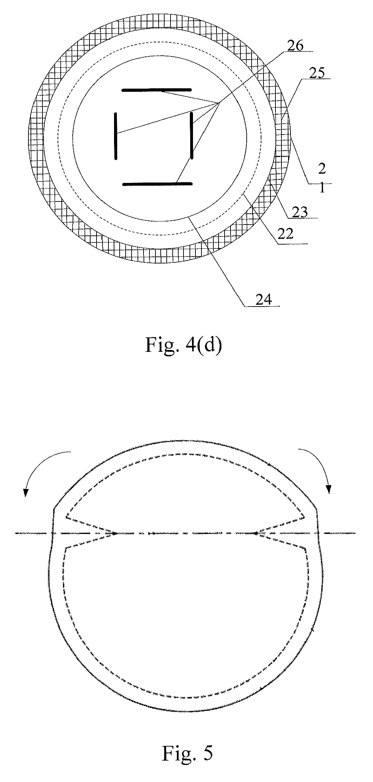

As shown in Figs. 4(a), 4(b), 4(c) and 4(d), another embodiment of the present application also discloses a breast pad. Similarly, the breast pad includes an isolation layer 21, an absorption layer 22 and a moisture permeable layer 23. The isolation layer 21, the absorption layer 22 and the moisture permeable layer 23 are arranged from bottom to top in sequence. A leakproof layer 24 is provided on the upper surface of the periphery of the moisture permeable layer 23.

Besides, the outer edge of the isolation layer 21, the outer edge of the moisture permeable layer 23 and the outer edge of a leakproof layer 24 are bonded together by hot pressing so as to form a bonded edge 25. There is no the absorption layer at the bonded edge. In addition, a storage space is formed by the leakproof layer 24 and the upper surface of the moisture permeable layer 23.

In this case, the leakproof layer 24 is a leakproof ring of 360 degrees, so as to prevent milk leakage from all angles.

Similar to the embodiment described above, at least one guiding channel 26 is further provided at the middle portion of the breast pad disclosed in this embodiment, and is formed by hot pressing. One or two double-sided stickers may be affixed on the outer surface of the isolation layer 21. In use, a stripping paper is torn off, and the breast pad is adhered to the brassiere. In addition, two surfaces of the absorption layer 22 are fixed onto the isolation layer 21 and the moisture permeable layer 23 respectively by adhesive.

Specifically, the guiding channel 26 disclosed in the embodiment may be implemented in various forms, generally in three forms as described below, and the number of the guiding channels 26 may be varied according to different requirements.

As shown in Fig. 4(a), two guiding channels 26 are arranged side by side vertically; as shown in Fig. 4(b), two guiding channels 26 are arranged side by side horizontally; as shown in Fig. 4(c), two guiding channels 26 are arranged side by side horizontally, and one guiding channels 26 is provided at a lower portion vertically; as shown in Fig. 4(d), two guiding channels 26 are arranged side by side both horizontally and vertically.

In the breast pad disclosed in the two embodiments described above, the moisture permeable layer is generally made of material with good air permeability, good water absorption and softness, and may effectively improve the comfort of the user. Specifically, the material for making the breast pad may be non-woven cloth having advantages such as environmental protection, moistureproof, breathable, soft, light, non-combustion, easy to decompose, non-toxic and non-irritation, and capable of being reused.

The absorption layer is generally made of material with strong absorption ability, for example, made of fluffy pulp or air-laid paper. The absorption layer may include two layers, with the upper layer being a fluffy pulp layer and the lower layer being a toilet paper layer, or with the upper layer being a mixed layer of fluffy pulp and macromolecule absorbers and the lower layer being a toilet paper layer.

The isolation layer is generally made of material with isolation function, for example, made of plastics.

The leakproof layer may be made of plastics. However, since the front face of the leakproof layer is in contact with the human body, the front face is made of super soft material in order to improve the comfort in use, while the back face is required to have leakproof property and thus is made of material with very strong leakproof property. Specifically, the leakproof layer may be a composite layer of non-woven cloth and plastic film, with the front face being the non-woven cloth and the back face being the plastic film. As such, the leakage may be effectively avoided, and the comfort of the user may be increased.

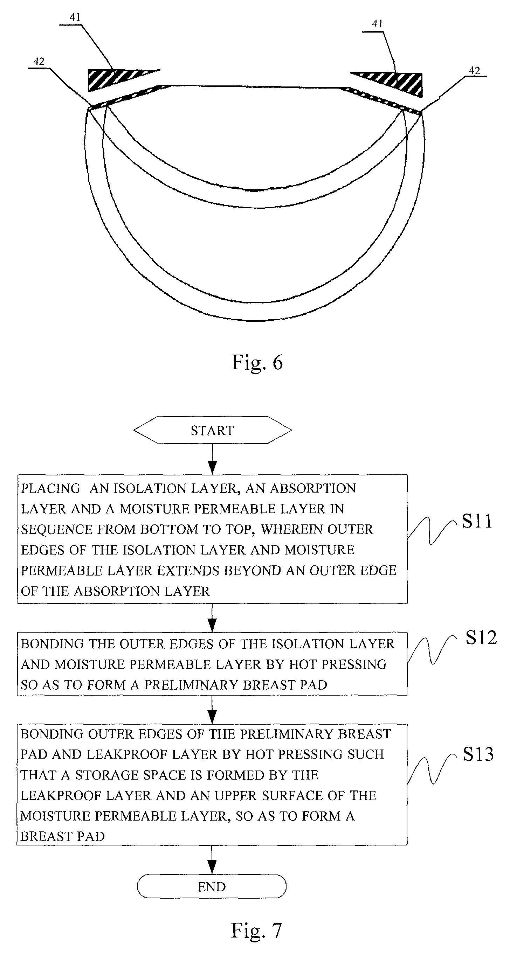

As shown in Figs. 5 and 6, the cup-shaped breast pad may be formed in the following way.

The breast pad disclosed in the above embodiments is placed flatwise; then, the breast pad is folded in the direction of arrow such that its upper and lower ends may be overlapped, and thus parts of the isolation layer and the moisture permeable layer are overlapped at the V-shaped notch; the overlapped isolation layer and moisture permeable layer are bonded integrally by hot pressing; redundant parts 41 are cut, but bonded edges 42 with a certain width still remain.

The processed cup-shaped breast pad is more adaptable to the brassiere, and fits against the brassiere fully so as to effectively absorb overflowed milk.

In conclusion, the breast pad disclosed in the embodiments of the present application is of a four-layer structure. In use, overflowed milk flows through the moisture permeable layer and is absorbed by the absorption layer. The isolation layer isolates the brassiere from the absorption layer, so as to prevent milk from contaminating clothing. Moreover, since a storage space is formed by the leakproof layer and the upper surface of the moisture permeable layer, overflowed milk may flow through the moisture permeable layer directly into the storage space when it flows downwardly under gravity, and may be absorbed gradually by the absorption layer without milk leakage.

Another embodiment of the present application further discloses a method for producing a breast pad, as shown in Fig. 7. The method includes the following steps.

In step S11, the isolation layer, the absorption layer and the moisture permeable layer are placed in sequence from bottom to top. The outer edges of the isolation layer and moisture permeable layer extend beyond the outer edge of the absorption layer.

Specifically, hygroscopic material is firstly processed into a cotton batting sheet, and the cotton batting sheet of hygroscopic material is directly processed into the absorption layer of desired shape by negative-pressure demould device.

When the cup-shaped breast pad is required to be produced, a V-shaped notch is required to be made at each of two opposite edges of the cotton batting sheet.

In step S12, the outer edges of the isolation layer and moisture permeable layer are bonded by hot pressing, so as to form a preliminary breast pad.

The outer edges of the isolation layer and moisture permeable layer are bonded by hot pressing so as to form a bonded edge. Since the outer edges of the isolation layer and moisture permeable layer extend beyond the outer edge of the absorption layer, there is no absorption layer at the bonded edge.

In step S13, the outer edges of the preliminary breast pad and leakproof layer are bonded by hot pressing, such that a storage space is formed by the leakproof layer and the upper surface of the moisture permeable layer, forming the breast pad.

Specifically, leakproof material may be compounded and shaped on line to form the leakproof layer, or the leakproof layer may be pre-produced for later use.

In practical production, as required, the bonded edge of the breast pad produced and shaped may be cut such that one bonded edge is left, as long as the width of the bonded edge left can ensure that the bonded portions cannot be separated one from another.

When the cup-shaped breast pad is to be produced, the breast pad produced is required to be placed flatwise; the breast pad is folded such that its upper and lower ends may be overlapped, and thus the isolation layer and the moisture permeable layer are overlapped; the overlapped isolation layer and moisture permeable layer at the periphery of each V-shaped notch of the absorption layer are bonded integrally by hot pressing, and cut off some parts thereof such that a bonded edge with a certain width still remains.

In order to guide the milk flow, the above method may further includes: forming at least one guiding channel on the moisture permeable layer of the breast pad processed and formed by hot pressing.

The arrangement of the guiding channel is the same as disclosed in the above breast pad embodiment, and is not described herein any more.

The various embodiments in the description are described in progressive manner, and each embodiment places emphasis on the differences between the embodiment and the other embodiments, with the same and similar points between the various embodiments being referred to each other.

The above description of the embodiments disclosed enables the person skilled in the art to practice and use the invention. Many modifications to these embodiments will be obvious for the person skilled in the art, and the general principle defined herein may be implemented in other embodiments without departing from the scope of the present application. Thus, the present application is not limited to these embodiments illustrated herein, but conforms to the broadest scope consistent with the principle and novel features disclosed herein.

## Claims

1. A breast pad comprising an isolation layer (11), an absorption layer (12), a moisture permeable layer (13), and a leakproof layer (14) in sequence from bottom to top, wherein the leakproof layer (14) is disposed on an upper surface of one end, or an upper surface of two opposite ends, or an upper surface of a periphery of the moisture permeable layer (13), and
wherein an outer edge of the isolation layer (11), an outer edge of the moisture permeable layer (13) and an outer edge of the leakproof layer (14) are bonded by hot pressing so as to form a bonded edge (15), the absorption layer (12) does not exist at the bonded edge (15), and a storage space is formed between the leakproof layer (14) and the upper surface of the moisture permeable layer (13).

2. The breast pad according to claim 1, wherein one or two double-sided stickers (17) are affixed to an outer surface of the isolation layer (11).

3. The breast pad according to claim 1, wherein two surfaces of the absorption layer (12) are adhered to the isolation layer (11) and the moisture permeable layer (13) by glue, respectively.

4. The breast pad according to claim 1, wherein the absorption layer (12) is a fluffy pulp layer, or an air-laid paper layer, or the combination of a toilet paper layer and a fluffy pulp layer in sequence from bottom to top, or the combination of a toilet paper layer and a mixed layer of fluffy pulp and macromolecule absorbers in sequence from bottom to top.

5. The breast pad according to claim 1, wherein the moisture permeable layer (13) is made of non-woven cloth.

6. The breast pad according to claim 1, wherein the leakproof layer (15) is a plastic film layer, or a composite layer of non-woven cloth and plastic film.

7. The breast pad according to claim 1, further comprising at least one guiding channel (16), wherein the at least one guiding channel (16) is formed by hot pressing.

8. The breast pad according to any one of claims 1 to 7, wherein the breast pad is in the shape of circle, square, triangle or sector, or is lace-shaped or cup-shaped.

9. A method for producing a breast pad comprising:
placing an isolation layer (11), an absorption layer (12) and a moisture permeable layer (13) in sequence from bottom to top, wherein outer edges of the isolation layer (11) and moisture permeable layer (13) extend beyond an outer edge of the absorption layer (12);
bonding the outer edges of the isolation layer (11) and moisture permeable layer (13) by hot pressing so as to form a preliminary breast pad; and
bonding outer edges of the preliminary breast pad and leakproof layer (14) by hot pressing such that a storage space is formed by the leakproof layer (14) and an upper surface of the moisture permeable layer (13), so as to form a breast pad.

10. The method according to claim 9, further comprising hot-pressing on the moisture permeable layer (13) of the breast pad to form at least one guiding channel (16).

## Patentansprüche

1. Brustpolster mit - in der Reihenfolge von unten nach oben - einer Isolationsschicht (11), einer Absorptionsschicht (12), einer Feuchtigkeits-durchlässigen Schicht (13) sowie einer auslaufsicheren Schicht (14), wobei die auslaufsichere Schicht (14) an einer oberen Oberfläche des einen Endes oder an einer oberen Oberfläche zweier gegenüberliegender Enden oder an einer oberen Oberfläche einer Peripherie der Feuchtigkeits-durchlässigen Schicht (13) aufgebracht ist, und
wobei ein äußerer Rand der Isolationsschicht (11), ein äußerer Rand der Feuchtigkeits-durchlässigen Schicht (13) und ein äußerer Rand der auslaufsicheren Schicht (14) durch Heißpressen verklebt sind, sodass sie einen verklebten Rand (15) bilden, wobei sich die Absorptionsschicht (12) nicht im verklebten Rand (15) befindet, und ein Stauraum zwischen der auslaufsicheren Schicht (14) und der oberen Oberfläche der Feuchtigkeits-durchlässigen Schicht (13) gebildet wird.

2. Brustpolster nach Anspruch 1, wobei einer oder zwei doppelseitige Aufkleber (17) an eine äußere Oberfläche der Isolationsschicht (11) angebracht sind.

3. Brustpolster nach Anspruch 1, wobei zwei Oberflächen der Absorptionsschicht (12) jeweils durch Kleben an die Isolationsschicht (11) und die Feuchtigkeits-durchlässige Schicht (13) angebracht sind.

4. Brustpolster nach Anspruch 1, wobei die Absorptionsschicht (12) eine Schicht aus lockerem Zellstoff oder eine Air-Laid-Papierschicht ist, oder die Kombination aus - in der Reihenfolge von unten nach oben - einer Toilettenpapierschicht und einer lockeren Zellstoffschicht, oder die Kombination aus - in der Reihenfolge von unten nach oben - einer Toilettenpapierschicht und einer gemischten Schicht aus lockerem Zellstoff und Makromolekül-Absorbern.

5. Brustpolster nach Anspruch 1, wobei die Feuchtigkeits-durchlässige Schicht (13) aus einem Vliesstoff hergestellt ist.

6. Brustpolster nach Anspruch 1, wobei die auslaufsichere Schicht (15) eine Folienschicht aus Kunststoff ist, oder eine Kompositschicht aus einem Vliesstoff und einer Kunststofffolie.

7. Brustpolster nach Anspruch 1, das ferner zumindest einen Führungskanal (16) aufweist, wobei der zumindest eine Führungskanal (16) durch Heißpressen gebildet ist.

8. Brustpolster nach einem der Anspruche 1 - 7, wobei das Brustpolster in Form eines Kreises, Rechtecks, Dreiecks oder Abschnitts ist, oder Spitzen-geformt oder Schalen-förmig ist.

9. Verfahren zur Herstellung eines Brustpolsters, das Folgendes aufweist:
Platzieren - in der Reihenfolge von unten nach oben - einer Isolationsschicht (11), einer Absorptionsschicht (12) und einer Feuchtigkeits-durchlässigen Schicht (13), wobei äußere Enden der Isolationsschicht (11) und der Feuchtigkeits-durchlässigen Schicht (13) über einen äußeren Rand der Absorptionsschicht (12) hinausragen;
Verkleben der äußeren Ränder der Isolationsschicht (11) und der Flüssigkeitsdurchlässigen Schicht (13) durch Heißpressen, sodass ein vorläufiges Brustpolster gebildet wird; und
Verkleben der äußeren Ränder des vorläufigen Brustpolsters und einer auslaufsicheren Schicht (14) durch Heißpressen, sodass ein Stauraum durch die auslaufsichere Schicht (14) und einer oberen Oberfläche der Feuchtigkeits-durchlässigen Schicht (13) gebildet wird, zur Bildung eines Brustpolsters.

10. Verfahren nach Anspruch 9, welches ferner ein Heißpressen auf der Feuchtigkeits-durchlässigen Schicht (13) auf dem Brustpolster umfasst, zur Bildung von zumindest einem Führungskanal (16).

## Revendications

1. Coussinet d'allaitement comprenant une couche d'isolation (11), une couche d'absorption (12), une couche perméable à l'humidité (13), et une couche étanche (14) en séquence de bas en haut, dans laquelle la couche étanche (14) est disposée sur une surface supérieure d'une extrémité, ou une surface supérieure de deux extrémités opposées, ou une surface supérieure d'une périphérie de la couche perméable à l'humidité (13), et
dans lequel un bord extérieur de la couche d'isolation (11), un bord extérieur de la couche perméable à l'humidité (13) et un bord extérieur de la couche étanche (14) sont collés par pressage à chaud de façon à former un bord collé (15), la couche d'absorption (12) n'existe pas au niveau du bord collé (15), et un espace de stockage est formé entre la couche étanche (14) et la surface supérieure de la couche perméable à l'humidité (13).

2. Coussinet d'allaitement selon la revendication 1, dans lequel un ou deux autocollants double face (17) sont fixés à une surface extérieure de la couche d'isolation (11).

3. Coussinet d'allaitement selon la revendication 1, dans lequel deux surfaces de la couche d'absorption (12) adhèrent à la couche d'isolation (11) et à la couche perméable à l'humidité (13) avec de la colle respectivement.

4. Coussinet d'allaitement selon la revendication 1, dans lequel la couche d'absorption (12) est une couche de pâte fluff, ou une couche de papier air-laid ou une combinaison d'une couche de papier toilette et d'une couche de pâte fluff en séquence de bas en haut, ou une combinaison d'une couche de papier toilette et d'une couche mixte de pâte fluff et d'absorbeurs macromoléculaires en séquence de bas en haut.

5. Coussinet d'allaitement selon la revendication 1, dans lequel la couche perméable à l'humidité (13) est faite d'une étoffe non tissée.

6. Coussinet d'allaitement selon la revendication 1, dans lequel la couche étanche (15) est une couche de film plastique, ou une couche composite d'étoffe non tissée et de film plastique.

7. Coussinet d'allaitement selon la revendication 1, comprenant en outre au moins un canal de guidage (16), dans lequel l'au moins un canal de guidage (16) est formé par pressage à chaud.

8. Coussinet d'allaitement selon l'une quelconque des revendications 1 à 7, lequel coussinet d'allaitement a la forme d'un cercle, d'un carré, d'un triangle ou d'un secteur, ou est en forme de dentelle ou en forme de coupelle.

9. Procédé pour produire un coussinet d'allaitement comprenant :
le placement d'une couche d'isolation (11), d'une couche d'absorption (12) et d'une couche perméable à l'humidité (13) en séquence de bas en haut, les bords extérieurs de la couche d'isolation (11) et de la couche perméable à l'humidité (13) s'étendant au-delà d'un bord extérieur de la couche d'absorption (12) ;
le collage des bords extérieurs de la couche d'isolation (11) et de la couche perméable à l'humidité (13) par pressage à chaud de façon à former un coussinet d'allaitement préliminaire ; et
le collage des bords extérieurs du tampon d'allaitement préliminaire et de la couche étanche (14) par pressage à chaud de façon qu'un espace de stockage soit formé par la couche étanche (14) et une surface supérieure de la couche perméable à l'humidité (13) de manière à former un coussinet d'allaitement.

10. Procédé selon la revendication 9, comprenant en outre un pressage à chaud sur la couche perméable à l'humidité (13) du coussinet d'allaitement de manière à former au moins un canal de guidage (16).
